Europäisches Patentamt

European Patent Office

Office européen des brevets

(11) Publication number: **0 373 771**
**A2**

(12) # EUROPEAN PATENT APPLICATION

(21) Application number: 89311874.5

(51) Int. Cl.5: **A61K 37/64**

(22) Date of filing: 16.11.89

(30) Priority: 18.11.88 JP 292227/88

(43) Date of publication of application:
20.06.90 Bulletin 90/25

(84) Designated Contracting States:
AT BE CH DE ES FR GB GR IT LI LU NL SE

(71) Applicant: TAISHO PHARMACEUTICAL CO.
LTD
24-1 Takata 3-chome Toshima-ku
Tokyo 171(JP)

Applicant: MITSUI TOATSU CHEMICALS, Inc.
2-5 Kasumigaseki 3-chome
Chiyoda-Ku Tokyo 100(JP)

(72) Inventor: Hanada, Kazunori
523-12, Haraichi
Ageo-shi(JP)
Inventor: Otomo, Susumu
2-20-5, Kamioinezuka
Konosu-shi(JP)
Inventor: Takeshita, Kimiyo
1246-22, Kawarabuki
Ageo-shi(JP)
Inventor: Higuchi, Shohei
516-41, Shimoishitoshimo
Kitamoto-shi(JP)
Inventor: Ike, Yoshimasa
750-7, Togo
Mobara-shi(JP)

(74) Representative: Lamb, John Baxter et al
MARKS & CLERK 57/60 Lincoln's Inn Fields
London WC2A 3LS(GB)

(54) New pharmaceutical uses for cystatins.

(57) The invention provides pharmaceutical compositions for the treatment of bone diseases and for the treatment of allergic diseases, which compositions contain a cystatin as active ingredient. The invention also provides for the use of cystatin in the manufacture of pharmaceutical compositions for the treatment of bone diseases and of allergic diseases. The invention further provides methods for the treatment of bone diseases or allergic diseases using cystatins.

EP 0 373 771 A2

## NEW PHARMACEUTICAL USES FOR CYSTATINS

The present invention relates to a new pharmaceutical use for a cystatin and more particularly, to use of a cystatin for the treatment of allergic diseases and bone diseases.

Cystatins are proteins present in animal and human cells and body fluids which specifically inhibit cysteine proteases. To date various kinds of cystatins have been isolated and their structures have been determined. Cystatin $\alpha$, cystatin $\beta$ and the like are known to function intracellularly and cystatin C or the like is known to be secreted and function extracellularly.

In more detail, Grubb et al. isolated cystatin C from cerebrospinal fluids as the first cystatin and determined its structure in 1982 [Proc. Natl. Acad. Sci. U.S.A., 79, 3024 (1982)]. Then, Katsunuma et al. isolated cystatin $\alpha$ from rat skin and determined its structure in 1983 [Biochem. Biophys. Res. Commun., 115, 902 (1983)] and in 1984, isolated cystatin $\beta$ from rat liver and determined its structure [Biochem. Biophys. Res. Commun., 121, 149 (1984)].

Since cystatins strongly inhibit the proteolytic activity of cysteine proteases, a possibility to use cystatins as drugs for treating various diseases induced by abnormal increase of cysteine proteases has been investigated. For example, a new cysteine protease has been found to be involved in the replication process of poliovirus in host cells and cystatins inhibit the cysteine protease, and thus a possibility of cystatins as viral infection inhibitors is under investigation [Proc. Natl. Acad. Sci. U.S.A., 83, 5392 (1986)]. A further possibility of using cystatins as drugs for treating amyotrophic diseases where a cystein protease abnormally increases is also suggested [Taisha (Metabolism), 24 (2), extra page 275 (1987)].

An object of the present invention is to provide a new pharmaceutical use of cystatins for the treatment of allergic diseases and bone diseases.

In one aspect of the present invention, there is provided a pharmaceutical composition for the treatment of bone diseases or allergic diseases comprising a cystatin as an active ingredient and a pharmaceutically acceptable carrier.

In another aspect of the present invention, there is provided a method for the treatment of bone diseases or allergic diseases which comprises administering a pharmaceutically effective amount of a cystatin to a mammal including a human being.

In further another aspect of the present invention, there is provided use of a cystatin for the manufacture of a pharmaceutical composition for the treatment of bone diseases or allergic diseases.

Cystatins used in the present invention may be any of intracellular type which localizes and exerts its effects intracellularly and extracellular type which exert its effects after extracellular secretion. Specific examples of the cystatins include intracellular type cystatins such as cystatin $\alpha$ (derived from rat), cystatin A (derived from human beings), cystatin $\beta$ (derived from rat), cystatin B (derived from human beings), etc.; extracellular type cystatin such as cystatin C (derived from human beings), cystatin S (derived from human beings), cystatin EW (derived from human beings), cystatin colostrum (derived from human beings), etc. Among them, human cystatins such as cystatin $\alpha$, cystatin A, cystatin B, cystatin C and the like are preferred. Cystatin C is one of the most preferred cystatins in the present invention since it has a property that it is readily incorporated into cells. These cystatins can be isolated from animal cells or body fluids such as sera, saliva, etc. [Proc. Natl. Acad. Sci. U.S.A., 79, 3024 (1982)]; Biochem. Biophys. Res. Commun., 115, 902 (1983); Protein Inhibitors, page 125, Japan Sci. Soc. Press and Springer-Verlag (1983); Cystein Proteinases and Their Inhibitors, page 497, Water de Gruyter & Co., Berlin (1986); FEBS Lett., 186, 41 (1985)]. These cystatins may also be recombinant cystatins produced by ordinary genetic engineering techniques. Examples of the recombinant cystatins include recombinant cystatin $\alpha$ (Japanese Patent Application No. 62-21706) and recombinant cystatin A (Japanese Patent Application No. 63-110402), which are obtained by chemically or biochemically synthesizing a gene encoding cystatin $\alpha$ or cystatin A, inserting the gene into an appropriate plasmid and expressing in E. coli.

The present inventors have revealed that cystatins significantly inhibit release of calcium from bone in experimental animal models such as osteoporosis, etc. and also strongly inhibit the production of IgE antibody in mice. Accordingly, cystatins are effective for the treatment of bone diseases such as osteoporosis, Behcet disease, hypercalcemia, osteomalacia, etc. Cystatins are also effective for the treatment of allergic diseases such as allergic skin disease, allergic rhinitis, allergic purpura, etc.

In any of the treatments of bone diseases and allergic diseases, cystatins may be administered parenterally, e.g., intravenously, intraarterially, intramuscularly, nasally, subcutaneously, intrarectally, etc., or orally.

Examples of parenteral preparations such as intravenous, intraarterial, intramuscular and subcutaneous preparations include injections, infusions, etc. These pharmaceutical preparations may be prepared in a

2

conventional manner. The injections, infusions, etc. may also be prepared by freeze-drying cystatin alone or together with carriers such as mannitol, lactose, albumin, etc. and charging the freeze-dried product into a vial, etc. Cystatins may also be prepared into liposomal preparations composed of a thin membrane of phospholipids such as lecithin, lysolecithin, sphingomyelin, etc. Cystatins may also be prepared into implanted type preparations which may be used by inserting subcutaneously and maintaining under the skin.

As the nasal preparations, there are powders comprising cystatins together with carriers such as cellulose lower alkyl ethers, e.g., hydroxypropyl cellulose, etc., polyacrylates e.g., sodium polyacrylate, etc., crystalline cellulose, etc.

The intrarectal preparations include suppositories. The suppositories may be prepared by thoroughly dispersing cystatins in a conventional base such as cacao butter, etc. in the presence of one or more surface active agents such as fatty acid esters, polyoxyethylene derivatives, pyridoxine fatty acid esters, sucrose fatty acid esters, phospholipids, etc. thereby to improve an absorption efficiency; and the like.

As the oral preparations, there may be exemplified the aforesaid liposomal preparations and the like.

In the case of using cystatins for allergic diseases, cystatins may also be applied in the form of sprays for spraying into the oral cavity or nasal cavity.

The preparations described above may all be prepared in a conventional manner. If necessary and desired, these preparations may further contain therein preservatives, color agents, stabilizers, etc. which are conventionally used in the art.

A dose of cystatins may vary depending upon age, body weight or conditions of patients or kind of cystatins to be administered, etc. but in the case of the treatment of bone diseased, may be generally in the range of from 0.1 to 500 mg/kg body weight/day, preferably 10 to 100 mg/kg body weight/day; in the case of the treatment of allergic diseases, generally in the range of from 0.1 to 500 mg/kg body weight/day, preferably 10 to 200 mg/kg body weight/day.

The pharmaceutical composition comprising cystatins as an active ingredient is extremely useful for the treatment of osteoporosis, Behcet disease, hypercalcemia, etc. Furthermore, the composition is also extremely useful for the treatment of allergic skin diseases, allergic rhinitis, etc.

The present invention will be described in more detail by referring to test examples and examples but is not deemed to be limited thereto.

Test Example 1

Activity on calcium release from bone:

Wistar male rats of 5 week age had been fed with low calcium content feed (about 0.02%, Standard A-Modified Assorted Feed manufactured by Oriental Yeast Co., Ltd.) for a week and then provided for the following test.

Cystatin α (which is a recombinant cystatin obtained by chemically and biochemically synthesizing a gene encoding cystatin α, inserting the gene into plasmid and transforming E. coli to express in E. coli; the N-terminal methionine is not acetylated: Japanese Patent Application No. 62-21706) was dissolved in 25 mM Tris-HCl buffer which had been made isotonic with NaCl. The solution was administered through the tail vein in a dose of 0.6 ml/100 g body weight.

Blood was collected over 3 hours after the administration of cystatin α, and serum calcium content was measured with Kit Calcium C-Test Wako for determination of calcium, manufactured by Wako Pure Chemical Industry Co., Ltd., according to the OCPC method [H. J. Gitleman, Anal. Biochem., 118, 421 (1967)].

The results are shown in Table 1.

Table 1

| Group | Number of Animal | Serum calcium content (mg/dl) Mean ± S.E. |
|---|---|---|
| Control | 7 | 8.64 ± 0.10 |
| Crystalin α: | | |
| 60 mg/kg body weight | 9 | 7.74 ± 0.20** |

** $P < 0.01$ (t-assay)

As is shown in Table 1, release of calcium from bone was significantly inhibited after administration of cystatin α in the dose of 60 mg/kg body weight.

Test Example 2

Activity on Production of IgE Antibody in Mice:

BDF$_1$ strain female mice of 8 week age were used for the test, with 7 to 10 mice for one group. Cystatin α as used in Test Example 1 was dissolved in a mixture of phosphate buffer and physiological saline and the resulting solution was used as a test chemical. A mixture of phosphate buffer and physiological saline containing no active ingredient was used as a control. The test chemical and the phosphate buffer physiological saline mixture were intraperitoneally administered to mice in different groups, independently.

The mice were sensitized by intraperitoneal administration of 4 mg of aluminum hydroxide adsorbed with 1 μg of ovalbumin thereto and at the same time, the test chemical was administered intraperitoneally to the sensitized mice. On Day 14 after the sensitization, blood was collected from the orbital vein of mice. After diluting the blood with physiological saline to 2-fold, the diluted blood was centrifuged and the resulting sera were made anti-sera to detect IgE antibody titers.

Furthermore, the same amount of antigen was intraperitoneally administered to the same group as booster on Day 25. On Day 41 after the sensitization, blood was collected in a similar method and the resulting sera were made anti-sera to detect IgE antibody titers.

Using the thus obtained sera, an amount of antibodies produced was determined in accordance with a passive cutaneous anaphylaxis (PCA) reaction method described in International Archives of Allergy and Applied Immunology, 48, 16 (1975) (Ovary, Z., Caiazza, S.S. and Kojima, S.: PCA Reactions with Mouse Antibodies in Mice and Rats).

That is, the collected sera were diluted to various concentrations and the diluted sera were intradermally injected to another rats (Wistar strain, male, weighing 200 to 250 g). Four hours after a solution of 4 mg of ovalbumin in 1 ml of 0.5% Evans blue physiological saline was intravenously administered to the rats and a serum threshold concentration for dye exudation was determined. The results are shown in Table 2.

4

Table 2

| Test Chemical | Number of Animal | IgE Antibody Titer of Anti-Ovalbumin/(mean ± S.E.) | IgE Antibody Titer of Anti-Ovalbumin/% of Control |
|---|---|---|---|
| Primary Response: | | | |
| Control | 10 | 2611 ± 988 | 100 (%) |
| Cystating α 200 mg/kg | 7 | 128 ± 34** | 4.9 |
| Secondary Response: | | | |
| Control | 10 | 2970 ± 883 | 100 |
| Cystating α 200 mg/kg | 7 | 585 ± 73** | 19.7 |

** $P < 0.01$ (Dunnet multiple analysis)

As is clear from the above results, cystatin significantly suppressed the production of IgE.

Test Example 3

Activity of Cystatin A on IgE Production System in Mice:

BDF₁ strain female mice of 8 week age were used for the test, with 8 mice for one group. Cystatin A was dissolved in physiological saline and the resulting solution was used as a test chemical. Physiological saline containing no active ingredient was used as a control.

The mice were sensitized by intraperitoneal administration of 4 mg of aluminum hydroxide adsorbed with 1 μg of ovalbumin thereto and at the same time, the test chemical was intravenously administered to the sensitized mice. On Day 14 after the sensitization, blood was collected from the orbital vein of mice. After diluting the blood with physiological saline to 2-fold, the diluted blood was centrifuged to give anti-sera. The resulting sera were treated in a manner similar to Test Example 2 to determine the amount of IgE antibody produced by PCA response. The results are shown in Table 3.

Table 3

| Test Chemical | Number of Animal | IgE Antibody Titer of Anti-Ovalbumin/(mean ± S.E.) | IgE Antibody Titer of Anti-Ovalbumin/% of Control |
|---|---|---|---|
| Control | 8 | 1920 ± 519 | 100 (%) |
| Cystatin A: | | | |
| 50 mg/kg | 8 | 1120 ± 227 | 58.3 |
| 100 mg/kg | 8 | 768 ± 97 | 40.0 |
| 200 mg/kg | 8 | 560 ± 11* | 29.2 |

** $P < 0.05$ (Dunnet multiple analysis)

As is clear from the above results, cystatin A significantly suppressed the production of IgE.

Test Example 4

<u>Toxicity:</u>

Cystatin $\alpha$ as used in Test Example 1 was administered intraperitoneally and intravenously to mice in a dose of 1 g per mouse but no change was noted in mice.

Example 1

Injections:

Freeze-dried cystatin $\alpha$ (600 g) was dissolved in 6 ml of physiological saline for injection. After aseptically filtering through a filter (pore diameter of 0.22 nm, manufactured by Japan Millipore Industry Co., Ltd.), 2 ml of the solution was charged in an ampoule and the space in the ampoule was substituted with nitrogen gas. The ampoule was sealed to give injections.

Example 2

<u>Liposomal Preparations:</u>

Yolk lecithin (110 $\mu$mol) and 50 $\mu$mol of cholesterol were dissolved in 10 ml of chloroform and the solution was charged in a flask of 100 ml. After thoroughly mixing, the solvent was distilled off on a hot bath of 40° C to form a thin layer on the inner wall of the flask. Drying in vacuum was performed in a desicator for further 3 hours. Cystatin A (30 $\mu$mol) was dissolved in 10 ml of physiological saline and the solution was charged in a flask as described above. The mixture was shaken on a vortexing mixer. After ultrasonication, the mixture was filtered through a filter having a pore diameter of 0.22 nm (manufactured by Japan Millipore Industry Co., Ltd.), 10 ml of the solution was charged in an ampoule and the space in the ampoule was substituted with nitrogen gas. The ampoule was sealed to give liposomal preparations for injection.

Example 3

<u>Suppositories:</u>

To 17 g of cacao butter was added 1.0 g of sodium glycolate and the mixture was thoroughly kneaded in a mortar. Cystatin $\alpha$ (2 g) was taken into the mortar and the base described above was gradually added thereto. The mixture was mixed to give a homogeneous suppository composition. The composition was charged in a container in an amount of 2 g to give suppositories.

Example 4

<u>Powders for nasal administration:</u>

Freeze-dried cystatin A (100 mg) and hydroxypropyl cellulose were taken and thoroughly mixed in a mortar to give a uniform powdery composition. The composition was charged in a capsule to give cystatin preparations for nasal administration.

**Claims**

1. A pharmaceutical composition for the treatment of bone diseases comprising a cystatin as an active ingredient and a pharmaceutically acceptable carrier.
2. A pharmaceutical composition according to claim 1 in which the cystatin is a human cystatin selected from cystatin α, cystatin A, cystatin B and cystatin C.

3. A pharmaceutical composition according to claim 1 in which the cystatin is cystatin α or cystatin C.

4. A pharmaceutical composition according to claim 1 in the form of a liposomal preparation.

5. A pharmaceutical composition for the treatment of allergic diseases comprising a cystatin as a active ingredient and a pharmaceutically acceptable carrier.

6. A pharmaceutical composition according to claim 5, having the characteristics defined in claim 2 or claim 4.

7. A method for the treatment of bone diseases which comprises administering a pharmaceutically effective amount of a cystatin to mammals including human beings.

8. A method according to claim 7 in which the cystatin is cystatin α or cycstatin C.

9. A method for the treatment of allergic diseases which comprises administering a pharmaceutically effective amount of a cystatin to mammals including human beings.

10. A method according to claim 7 or claim 9 in which the cystatin is a human cystatin selected from cycstatin α, cystatin A, cystatin B and cystatin C.

11. A method according to claim 7 or claim 9 in which the cystatin is administered in a dose of 0.1 to 500 mg/kg bodyweight/day.

12. A method according to claim 7 or claim 9 in which the cystatin is administered in the form of a liposomal preparation.

13. The use of a cystatin for the manufacture of a pharmaceutical composition for the treatment of bone diseases.

14. The use according to claim 13 in which the cystatin is cystatin α or cystatin C.

15. The use according to claim 17 in which the composition is for the treatment of osteoporosis, Bechet disease or hypercalcemia.

16. The use of a cystatin for the manufacture of a pharmaceutical composition for the treatment of allergic diseases.

17. The use according to claim 13 or claim 16 in which the cystatin is a human cystatin selected from cystatin α, cystatin A, cystatin B and cystatin C.

18. The use according to claim 13 or claim 16 in which the composition is in the form of a liposomal preparation.

19. The use according to claim 16 in which the composition is for the treatment of allergic skin diseases, allergic rhinitis or allergic purpura.